# EUROPEAN PATENT SPECIFICATION

(11) **EP 1 213 989 B1**
(45) Date of publication and mention of the grant of the patent: **07.09.2005**
(21) Application number: 00936819.2
(22) Date of filing: 29.05.2000
(51) Int. Cl.: A61B 1/32

(54) **SET OF ACCESSORIES FOR TRANSANAL OPERATIONS**
INSTRUMENTENSATZ FÜR TRANSANALE EINGRIFFE
ENSEMBLE D'ACCESSOIRES POUR OPERATIONS TRANSANALES

(30) Priority: 20.09.1999 EP 99830591
(43) Date of publication of application: 19.06.2002
(73) Proprietor: Sapi Med S.p.A., 15100 Alessandria (IT)
(72) Inventor: ODDENINO, Gian, Paolo, I-15100 Alessandria (IT)
(74) Representative: Siniscalco, Fabio
(86) International application number: PCT/EP2000/004865
(87) International publication number: WO 2001/021060

(56) References cited:
- DE-A- 19 739 033
- DE-C- 830 544
- US-A- 4 819 620
- US-A- 4 834 067
- US-A- 5 003 962
- US-A- 5 249 568
- US-A- 5 570 692
- US-A- 5 716 329

## Description

The subject of the present invention is a set of accessories for transanal operations.

In particular, reference will be made in a non-limiting way, to the use of said set in an operation for the removal of a cylindrical or tubular section of mucosa from a rectal ampule.

It is known that this operation is performed in order to remove from the rectal ampule the tubular section of mucosa in which the blood vessels that feed a haemorrhoidal prolapse run. In order to prepare this operation it is necessary to realize, at a fixed depth of the rectal ampule, a purse-string suture in the mucosa wall or the mucosa. Once the purse-string suture has been created it is possible to introduce an intraluminal anastomotic surgical stapler and, after having received the purse-string suture on the operative extremity of the surgical instrument, remove the mucosa grasped by the instrument and forming said tubular section, simultaneously suturing the remaining flaps.

At present, in order to carry out said purse-string suture, it is possible to use a potentially traumatic steel inserter. Despite the use of the inserter, the field of operation and the visual field are very limited. Due to this problem at present the realization of a purse-string suture is particularly difficult. First of all the evaluation of the position of the suture is approximate, in other words it is difficult to perceive the depth of the operation correctly. This difficulty does not make it possible to create a suture which is regular for the whole of its length. Moreover, the need to use the known inserter during all stages of the operation is extremely traumatic. The trauma caused by the operation is increased by the fact that in order to place each stitch in the suture of the purse-string suture it is necessary to grasp and isolate each single portion of mucosa from the wall.

DE-C-830 544 discloses an instrument comprising the features of the preamble of claim 1.

The problem on which the present invention is based is that of proposing a set of accessories for transanal operations, which has structural and functional characteristics capable of overcoming the above-mentioned difficulties quoted with reference to the technical note.

The invention is as defined in the appended claims.

In accordance with the present invention, at least one valve with an active portion having an opening to receive the introflexed part of the mucosa is provided.

Also in accordance with the present invention, a set of accessories for a transanal operation aimed at resolving said problem is characterized by including at least one spatula having an active portion which joins a handle at an angle.

In order to better understand the invention, one of its embodiments, which is in no way limiting, is described below and illustrated in the enclosed drawings, in which:
Figure 1 shows a top view of a first accessory according to the present invention;
Figure 2 shows a front view of the accessory in Figure 1;
Figure 3 shows a perspective view of the accessory in Figure 1;
Figure 4 shows a perspective view of a second accessory to be used in combination with the first accessory;
Figure 5 shows a lateral view of the accessory in Figure 4;
Figure 6 shows a perspective view of a third accessory;
Figure 7 shows a lateral section of the accessory in Figure 6;
Figure 7a shows a lateral section of the detail of Figure 7, marked by the arrow VIIa;
Figure 8 shows a perspective view of the accessory in Figures 1 to 3 during an initial stage of an operation in a section of rectal ampule ;
Figure 9 shows a partially sectioned perspective view of the section of rectal ampule as in Figure 8 during a second stage of the operation;
Figures 10, 11 and 12 show perspective views of successive stages of use of the accessory in Figures 6 and 7, co-operating with an intraluminal anastomatic surgical stapler ;
Figure 13 shows a cross section of a third stage of the operation in the intestinal tract;
Figure 14 shows a cross section of a further stage of the operation in the section of rectal ampule and
Figure 15 shows a sectioned perspective view of the section of rectal ampule after the operation.

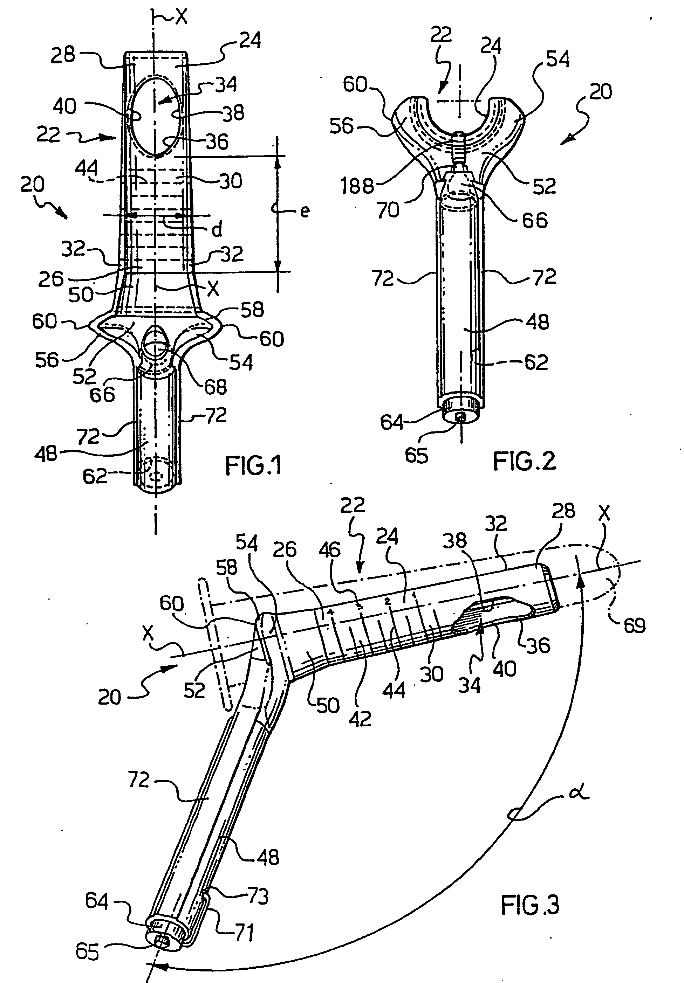

With reference to the above Figures, number 20 globally indicates a first accessory to be used in the preparation stages for an operation to be effected using a transanal access surgical instrument. Said accessory includes a valve 20. Said valve 20 has an active portion 22 which is douche shaped or, in other words, rounded, or in yet other words, channelled 24 on axis X - X, which has one side convex (back) and one side concave. Said channel 24 has a semi-circular cross section. According to a preferred version of the invention, said channel 24 is tapered to a first extremity 26 and a second extremity 28. The cross section diminishes in diameter or width "d" passing through a first and a second extremity 26, 28. According to a further version of the invention, the wall 30 of said channel 24 diminishes in thickness passing from the first to the second of said extremities 26 and 28. The borders 32 of the channel 24 are rounded, in order to eliminate sharp edges (Figures 1 and 3).

In proximity to said second extremity 28, in the wall 30 of the valve 20 an opening or window 34 is foreseen. Said window 34 is positioned centrally to the channel 24 and has a rounded edge 36. The edge 36 of said window 34 has a generically drawn out shape, for example elliptical, or, advantageously, it is polygonal or, with a further advantage, it is rhomboidal. The edges of the polygonal window 34 are blended or rounded. Said window 34 is extended principally in a longitudinal direction with respect to the valve 20, according to the direction of the axis X-X. Preferably, the polygonal edge 36 of the window 34 shows two opposing areas or sections 38 and 40 arranged parallely and longitudinally to the channel 24. Said window 34 is foreseen at a set distance "e" from the first extremity 26 of the channel 24. The external surface 42 of the back of the channel preferably shows a graduated scale 44. Said graduated scale 44 is positioned in the section of the channel which separates the window 34 from the first extremity 26. Said graduated scale 44 is flanked by numerical indications 46 showing the distance from the edge 36 of the window 34. For example, said graduated scale 44 and said numerical indications 46 are in relief on the wall 30 of the valve 20.

The first extremity 26 of the channel 24 is connected with a handle or a grip 48. The axis of said grip 48 forms an angle "α" with the active portion 22 to channel 24. Thus the grip 48 is distanced from the X - X axis of the active portion 22. For example, the grip 48 forms an angle "α" with the active portion 22 between 100 degrees and 145 degrees and, preferably equal to 120 degrees. According to a particular form of realization, said extremity 26 joins a section of coaxially positioned conic channel 50. Said conic section 50 is connected with a section of tapered channel 52 which is in turn connected to the grip 48 passing through a section that splays into two wings 54 and 56 or, in other words, a stretch in which channel of the funnel-shaped section 52 reaches its maximum expansion to then close suddenly on the grip 48. The edge 58 of the channel of the funnel-shaped section 52, in the point at which the widened section shows the sudden variation in width, has a point 60 or guard for the valve 20.

The grip 48 can be associated with a means of illumination 64. According to a preferred version of the invention, the grip 48 is cylindrical and housed by a seat 62. Said seat 62 accepts removably said means of illumination 64 (removable means). Preferably the grip 48 is tubular and the cavity of said tubular grip forms the seat 62 for the means of illumination 64. Said seat 62 has a tapered or truncated conical terminal section 66, with a gradually decreasing section or opening. Said tapered section 66 ends in a circular opening 68, in correspondence with the tapered channel 52 and in proximity of the first extremity 26 of the active portion 22. The truncated conical surface of the tapered extremity 66 is the contact surface. For example, said seat 62 houses a light source or pencil light 64 or pen light, of known type, fitted with an on/off button 65 and with a light bulb 70 poking out of the circular opening 68. The pencil light source 64 is locked in the working position by hooks housed in a seat in the grip. For example the means of illumination 64 have an elastic extension 71 fitted with a protuberance or terminal stalk, which can be clipped into the seat 73 foreseen on the outside of the wall of the grip 48. Nerves 72 extend longitudinally and externally along the sides of the grip 48, uniting on the edge 58 of the section of tapered channel 52 (Figures 2 and 3).

The valve 20 is in biocompatible synthetic material and, preferably, in plastic for foodstuffs, for example of the type defined by the Italian Ministerial Decree dated 21.03.1973. A suitable material is for example the polystyrene ARL, preferably transparent. Said valve is constructed for example by injection moulding.

The valve 20 can be associated with a cylindrical or conical inserter 69 of known type. Said inserter 69 has a pointed or ogival extremity which, in a position associated with the valve 20, extends beyond the second extremity 28 of the channel 24 for atraumatic introduction into the anal orifice.

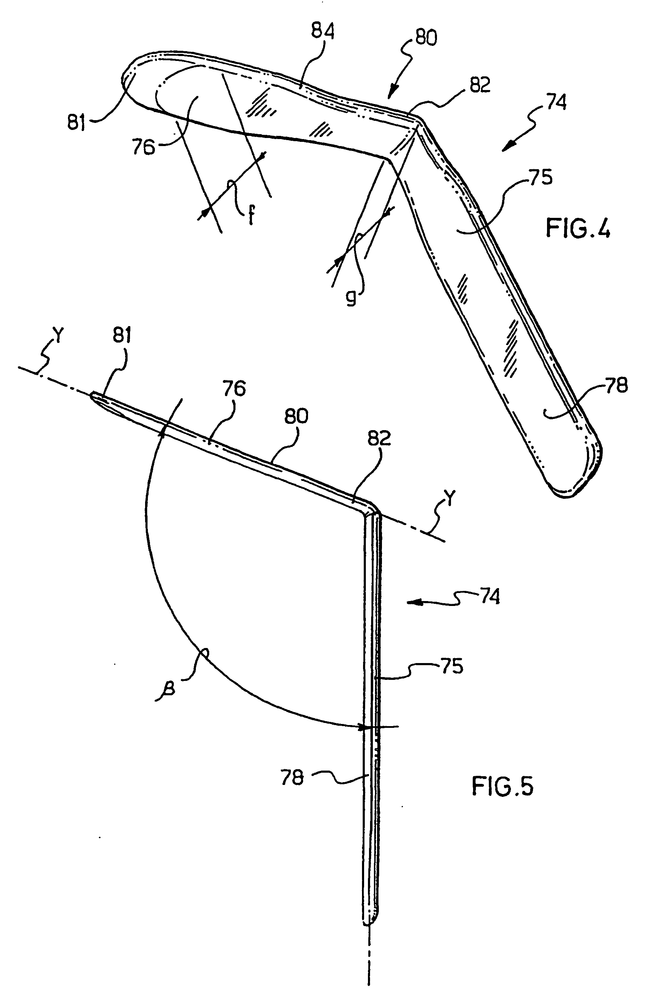

In the drawings it is also possible to note a second accessory, to be used in combination with the first accessory. Said accessory includes a depressor or spatula 74. Said spatula 74 consists of a blade 75 with an active portion 76, on axis Y - Y, joining at an angle "β" with a portion of the handle or grip 78. Said grip 78 is preferably shaped ergonomically. The angle "β" between the active portion 76 and the grip 78 distances the grip 78 from the axis Y - Y of the active part 76. For example the angle "β" varies between 100 degrees and 140 degrees and is preferably equal to 120 degrees. Said blade 75 has a slightly curved transverse section, for example, a circular arch, with concavity turned externally to the angle "β" marked by the spatula 74 or, in other words, it presents a curve on the outer edge 80 of the spatula 74 or concave side. In other words, the active portion 76 of said spatula 74 is channelled. The active portion 76 tapers towards the free extremity 81. Said active portion 76 has a width superior to the central section 82 which joins the grip 78. For example, said active portion 76 has a width "f" approximately equal to the length "d" of the active portion 22 of the valve 20 and said central section 82 has a width "g" equal to the minimum value sufficient to resist the stresses produced when the spatula 74 is used, while at the same time being of limited bulk. The spatula 74 has rounded edges 84, to avoid sharp edges. Said spatula 74 is in synthetic biocompatible material. For example, the spatula is in plastic for foodstuffs and, preferably, is in transparent ARL polystyrene (Figures 4 and 5).

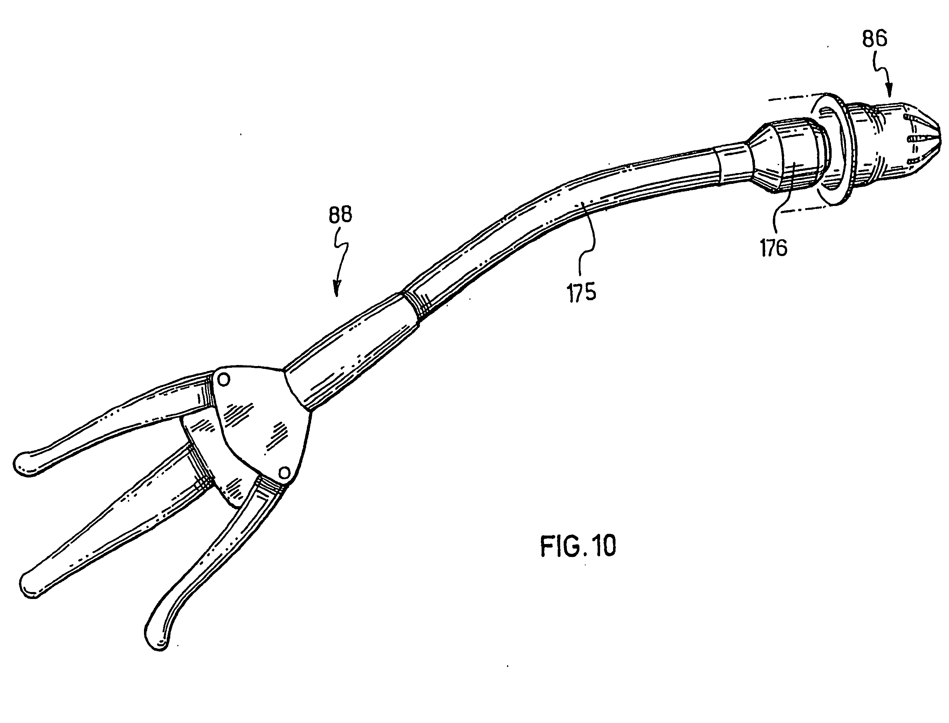

In the drawings it is also possible to note a third accessory not forming part of the present invention, which includes a mandrel or inserter 86. Said inserter 86 co-operates with a intraluminal anastomatic surgical stapler 88 or circular stapler, in a manner which will be described in detail below (Figure 10). Said inserter 86 is tubular sleeve shaped 90, on axis Z - Z, with a tapered section 92. The sleeve 90 has an initial section or lead-in 96 connected to a second section or annular section 98 with a smaller cross section at one free extremity 94. Said annular section 98 has an axial extension "h" variable between 10 mm and 15 mm and, preferably, equal to 13.3 mm and said lead-in 96 has an axial extension "i" variable between 8 mm and 12 mm and preferably equal to 10 mm. Said lead-in 96 and said annular section 98 are slightly conical towards the tapered section 92 and are connected by a truncated conical linking section 100. For example, the internal surface 102, 104 of the lead-in 96 and of the annular section 98 has a degree of taper "χ" variable between 0.15 degrees and 0.5 degrees and preferably equal to 0.25 degrees. The linking section 100 has a degree of taper "δ" variable between 3 degrees and 7 degrees and preferably equal to 5 degrees. Said truncated conical linking section 100 has, for example, an axial extension "1" variable between 4 mm and 8 mm and preferably equal to 6 mm. Said lead-in 96 has at the free extremity 94 a border 106 positioned transversely to the axis Z - Z of the sleeve 90. Inside the lead-in 96 there are annular ridges 108. Said ridges are elastically deformable on the axis Z - Z. Preferably, said ridges 108 have a sliding surface 110 on the extremity turned towards the axis Z - Z of the sleeve 90. Advantageously, said sliding surface 110 is positioned at a distance "m" from the axis of symmetry Z - Z of the sleeve 90 equal to the maximum distance "m" at which the internal surface 104 of the annular section 98 is positioned.

The tapered section 92 of said sleeve 90 is flower-shaped. Said flaps 112 of said flower shape have an internal surface 113 and an external surface 115 and overhang the annular section 98. The flaps 112 are elastically deformable in a direction radial to the sleeve 90 from a closed position, a bundle of converging flaps 112, to an open position, a bundle of parallel flaps 112. In particular, said flaps 112 have a rectilinear terminal section 114 joined by a curved section 116, forming, with the flaps 112 in the closed position, a truncated cone mantle which reduces the lumen "n" at the extremity opposite the lead-in 96 of the sleeve 90 to a value of less than half of the lumen "o" of the lead-in 96. For example, said lead-in 96 has a maximum diameter "o" variable between 30 mm and 40 mm and preferably equal to 35.5 mm and said minimum lumen formed by the closed flaps 112 has a diameter "n" variable between 10 mm and 20 mm and preferably equal to 15 mm. Consequently the flower-shaped section has conicity "ε" preferably equal to 30 degrees. Said flaps 112, even when in a closed position, have contiguous edges 118 which do not meet, creating longitudinal divisions 120. For example, the distance between two contiguous edges 118 of the flaps 112 in the closed position varies between 0.5 mm and 1 mm and is preferably equal to 0.87 mm. The extremity 122 of said flaps 112 has a rounded edge 124, and the contiguous or lateral edges 118 of the flaps 112 are rounded in correspondence with the external surface of the sleeve 90. In the closed position before use the flaps 112 are connected by bridges 128. For example, inside the flower-shaped section there is at least one ring 130, 132, and 134 which connects the flaps in the closed position. Preferably, said at least one ring is in fact three rings 130, 132, 134 positioned transversely to the axis Z - Z of the sleeve 90 at various heights of the flaps 112. A first ring 130 is positioned in proximity with the free extremity 122 of the flaps 112, a second ring 132 in proximity with the base of the flaps 112 where they are attached to the annular section 98 and a third ring 134 in correspondence with the middle of the flaps 112. Said rings 130, 132 and 134 have weak points 136 in correspondence with only one of the lateral contiguous edges 118 of the flaps 112. Said weak points 136 are constructed so that the bridges 128 will not break when the flaps 112 are strained by an action radial to the axis Z - Z of the sleeve 90. Said bridges 128 give way in correspondence with only one of the lateral contiguous edges 118 of the flaps 112, when subjected to a relatively low force radially directed towards the outside of the sleeve 90. In particular, said bridges 128 give way when they are forced by the passage of a surgical instrument, such as a intraluminal anastomatic surgical stapler 88, inside an inserter 86. Said rings 130, 132 and 134 have, for example, a trapezoidal transverse section 138 whose base 140 is fixed to the internal surface 113 of the flaps 112 and has an oblique side positioned transversely to the axis Z - Z of the sleeve 90. Said rings 130, 132 and 134 have, for example, a section 138 at height "p" variable between 0.5 mm and 0.9 mm and preferably equal to 0.7 mm and a width "q" variable between 0.07 mm and 0.47 mm and preferably equal to 0.27 mm.

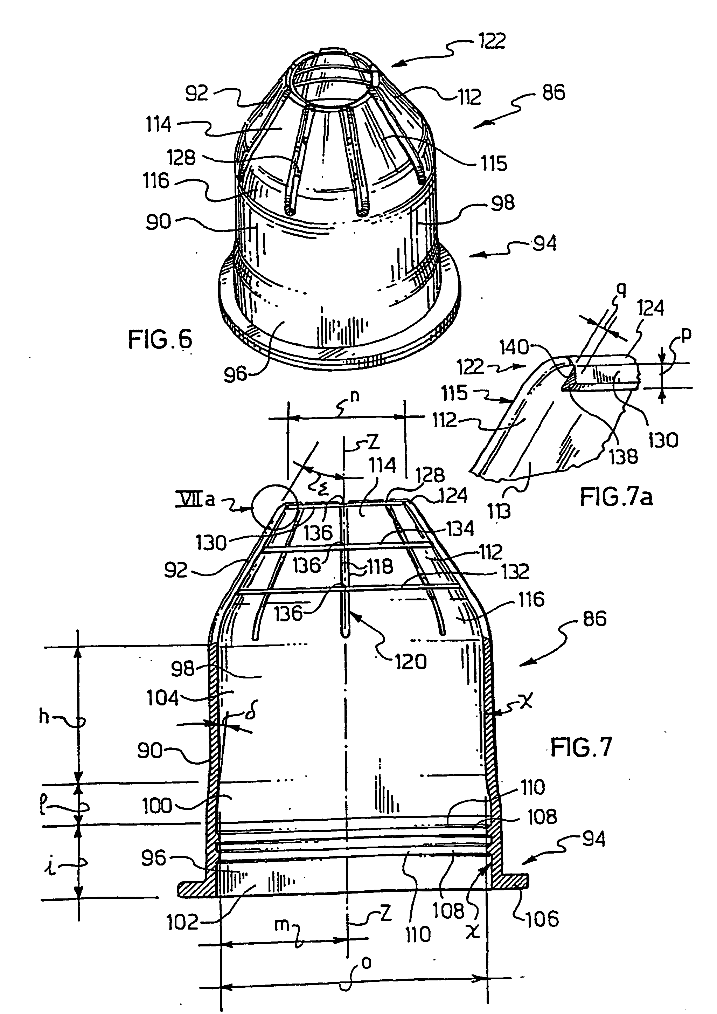

Said inserter 86 is in biocompatible synthetic material, for example, in polyethylene and, preferably, in mid-density polyethylene for foodstuffs and is constructed, for example, by injection moulding. (Figures 6, 7 and 7a).

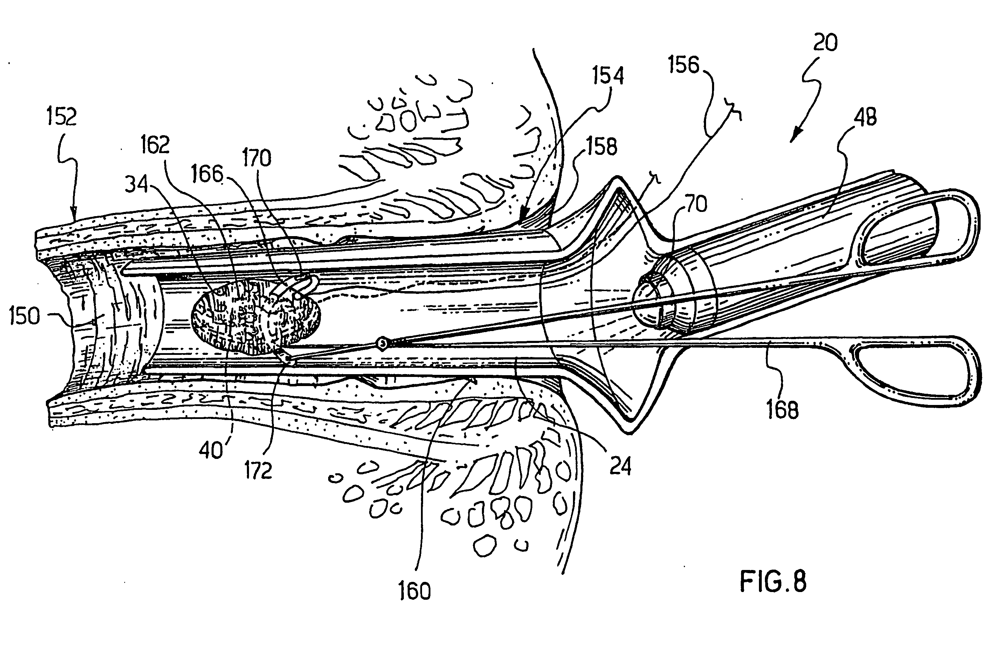

The method for using the above-described accessories 20, 74 and 86 is described below, taking as a non-limiting example of an operation to remove a section of mucosa 150 of the rectal ampule 152 in proximity of the anal duct 154, with reference to Figures 8 to 15.

If, for example, prolapsed haemorrhoids are present, one of the operative techniques consists of removing a tubular or cylindrical section of mucosa of the rectal ampule. Said section must be removed at a set depth on the rectal ampule or, in other words, at a suitable distance from the dentate line, for example at approximately 60 mm from the anal orifice.

This operation comprises the following principal stages. First of all, thanks to the fact that the mucosa can slide freely on the submucosal layer, a ring or circumferential purse-string suture is created at said depth of the mucosa. Successively the purse-string suture is closed, strangling the mucosa cylinder, which positions itself transversely to the axis of the rectal ampule. The mucosa cylinder deformed to a disc or ring shape, is then resected, simultaneously suturing the remaining overlapped rims of the mucosa.

The various surgical steps are described in more detail below.

First of all, some stages of the preparatory stages are described. First the ogival inserter 69 and the valve 20 are disconnected. Then a suture thread 156 is passed through the window 34 on the valve 20 and held across the window 34. Said suture thread 156 is passed from the convex side to the concave side of the valve 20, leaving a section of approximately 200 mm outside the valve 20. The internal portion of the suture thread 156 is positioned along the concavity of the valve 20 and then along the grip 48 of the same. The ogival inserter 69 is then repositioned in the channel 24 of the valve 20. A pencil light 64 is introduced into the seat 62 of the grip 48 until it sits against the surface of the tapered end 66, allowing the bulb 70 to poke out of the upper circular opening 68.

The stages of the operation are described below. The valve 20, fitted with the ogival inserter 69, is introduced into the anal duct 154 as far as possible, so that the valve guard 20, that is the funnel-shaped section 52, is in contact with the perineal tissue surrounding the anus 158. The ogival inserter is then removed 69. Once the desired position is reached, thanks to the positioning of the bulb 70 of the light 64 in proximity with the first extremity 26 of the channel 24, it is possible to attain perfect illumination of the operative area and at the same time free access, not obstructed by the means of illumination 64. Access to the operative area is particularly facilitated by the wings 54 and 56 of the funnel-shaped section 52, which hold the section of anal duct 154 apart. Once the ogival inserter 69 has been removed the spatula 74 which holds the mucosa 150 opposite the valve 20 away from the operative field is introduced into the rectal ampule. The position of the grips 48 and 78 of the valve 20 and the spatula 74 at an angle with respect to the relative active portions 22 and 76, makes it possible to maintain the active portions 22, 76 in the correct position, manipulating the accessories outside the visual field. Once the external section of the suture thread 156 is fixed to the operative field, one proceeds with light movements of the handle 48 of the valve 20, causing an extrusion of rectal mucosa 150 in the window 34 at a suitable distance from the dentate line 160. To evaluate whether the window 34 is positioned at the correct depth, it is possible to use the graduated scale 44 on the valve 20. When the mucosa 150 is seen to be extruded or introflexed through the window 34 of the valve 20, a portion of mucosa 162 is integrally transfixed with a suture stitch 164 from right to left, using a suture thread 156 fitted with a rounded needle 166, commonly known as atraumatic needle This operation is carried out by manoeuvring the needle 166 with the needle holder 168 of known type, in proximity with its eye 170. The left portion of the channel 24 of which the valve 20 is constructed, and therefore the edge or border 40 marking the window 34 itself, are used as a rest for the tip 172 of the needle 166 which pokes out of the transfixed portion of mucosa 162. In this way, the point 172 of the needle 166 is easily identifiable and can be retracted using the needle holder 168 (Figure 8). The suturing procedure is then completed, with the suture thread 156 still across the window 34 of the valve 20, for the entire circumference of the rectal ampule 152, moving the valve 20 at an angle and the counterpoised spatula 74 at an angle sufficient to cause a new portion of mucosa of the circumferential section of the rectal ampule 152 to extrude into the window 34 at the same depth. Once the suturing is completed, the valve 20 and the spatula 74 are removed and a digital check is made of the circumferential completeness of the purse-string suture 174 (Figure 9). This stage of the operation is completed by cutting the needle 166 and blocking the two ends of the suture thread 156 with a clip.

The stages relative to the preparation and use of the intraluminal anastomatic surgical stapler 88 are described below. Once the purse-string suture 174 is completed, it is necessary to introduce the surgical instruments necessary for the removal of the section of mucosa adjacent to the purse-string suture 174 and for the suturing of the remaining circumferential flaps. Said operation is carried out using a intraluminal anastomatic surgical stapler 88, of known type, which has at the operative extremity a stalk 175, an anvil 176 housing a cylindrical blade 178 and a device for shooting in the clips 180. A disc or head 182 co-operates with said anvil 176 shaped ogivally at the top and controlled by means of a jointed rod 184, or control rod, distancing (open position) and approaching (closed position) the anvil 176, in order to close the clips 180 during shooting or introduction of the clips in the tissues. Said anvil 176 and said head 182 form the cutter of the suturing device 88.

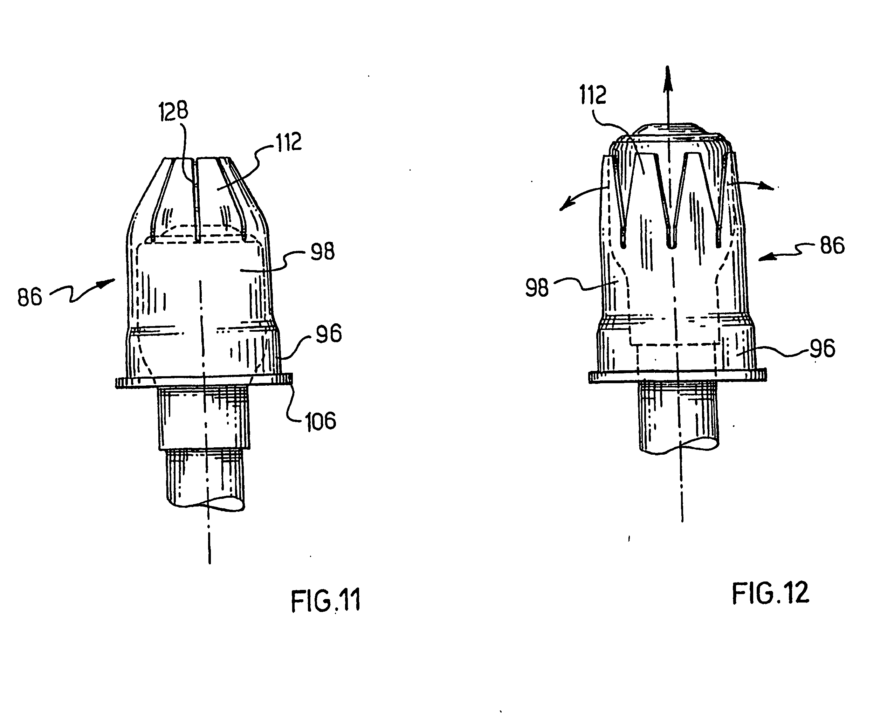

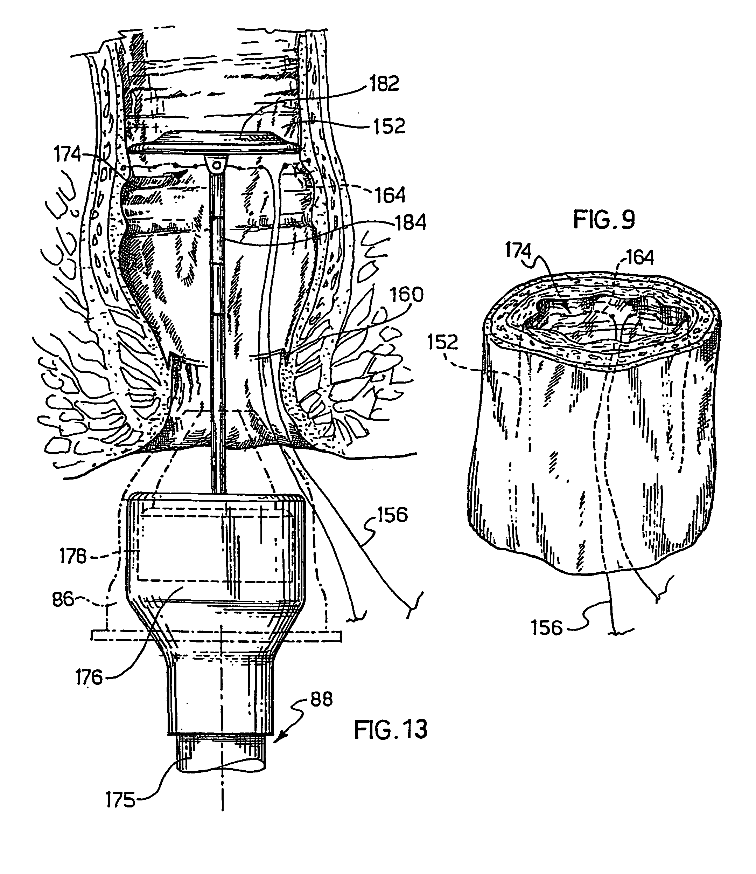

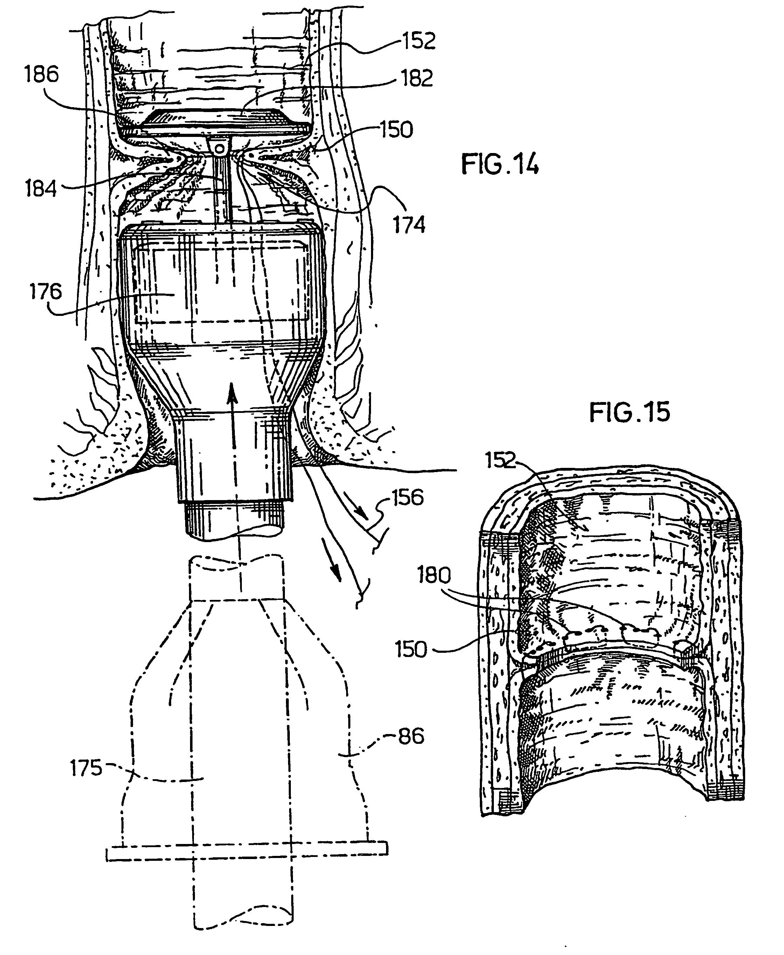

First of all, the suturing device 88 is opened completely and the head 182 is removed. Then the flower-shaped inserter 86 is fitted on the anvil 176 of the suturing device 88. The head 182 is repositioned, the suturing device 88 being opened as far as possible. The head 182 is introduced into the anal duct 154, ensuring that the head 182 overcomes the obstacle of the purse-string suture 174 previously stitched (Figure 13). The two ends of the suture thread 156 are knotted and a check is made to ensure that the mucosa 150 is positioned around the control rod 184 of the suturing device 88. In particular, on tightening the purse-string 174 around the stalk of the control rod 184, the mucosa 150 will stick around the stalk 184. In other words, the mucosa 150 will be introflexed positioning itself in such a way as to form an annular disc 186. To do this the ends of the suture thread 156 are pulled and knotted until they are in a strangulated position around the control rod 184 (Figure 14). Later it is necessary to bring the anvil 176 in proximity with the head 182. To do this it is necessary to introduce the anvil into the anal duct 154. Since the anvil 176 does not have a rounded ogival form, it is possible that during this operation the very delicate epithelium of the anus or the perineal tissue surrounding the anus may be damaged. Therefore the tapered section 92 and the annular section 98 of the inserter 86 are inserted into the anal duct. Thanks to the bridges 128 this operation is carried out without deforming the tapered section 92, avoiding disordering of the gathered position of the perfectly truncated conical flaps 112. It is then possible to tighten the cutting of the suturing device 88. The anvil 176 breaks the bridges 128 and passes through the flaps 112 moving into the rectal ampule 152. The anvil 176 is placed beside the head 182 tightening the annular disc 186 of mucosa 150 (Figures 12 and 14).

Once the cutting of the suturing device 88 has been tightened, the flower-shaped inserter 86 is withdrawn from the anal duct 154 and positioned on the stalk 175 of the suturing device 88.

Successively the annular disc 186 is cut withdrawing the cylindrical blade 178 and simultaneously shooting metal clips 180 into the rims or borders folded and laid against the remaining mucosa 150 (Figure 15).

Once the suturing has been carried out the suturing device 88 is removed and the rim of the suturing is checked, positioning, by means of the ogival inserter 69, the valve 20 with the window 34 in correspondence with the suture itself, rotating it as necessary. Finally the valve 20 is removed.

It is clear that variations and/or additions may be made to the procedures described and illustrated above.

The grip 48 of the valve 20 may be constructed as a channel, equipped with ridges for a notch coupling with the means of illumination 64.

At the extremity of the grip 48, facing the circular opening 68 housing the bulb 70 of the light, it is possible to foresee guides for the beam of light, capable of orienting said beam according to the axis X - X of the channel 24. Said means of illumination are for example formed of a bar to guide the light 188 (Figure 2). Said bar 188 is curved and conveys the light from the bulb 70 of the light on the first extremity 26 of the channel 24. Said bar 188 is part of the body of the valve 20. In this case the ogival inserter 69 which can be associated is fitted with a channel that, in the working position, holds the bar 188.

With reference to the inserter, the length of the annular section can vary according to the depth at which the operative extremity of the surgical instrument is positioned. In this way the non-rounded extremity of the instrument does not damage the section of intestinal tract to be passed through. During introduction of the inserter the truncated cone tapered section gradually and atraumatically widens the wall of the intestinal tract to be passed through.

All the accessories described are disposable and are therefore used for only one operation.

As will be appreciated from the above the valve which is the subject of the present invention makes it possible to create a perfectly regular purse-string suture at a set depth of the rectal ampule, guaranteeing optimal preparation of the mucosa section for its removal. Thanks to the window in the valve, the mucosa introflexes spontaneously, avoiding a traumatic grip. The correct distance from the dentate line at which the mucosa grasps the mucosa to create the purse-string suture is one of the most important aspects of the operation. Using the proposed valve it is possible to measure the point at which to grasp the mucosa prolapse with great precision, avoiding approximate procedures.

In the valve, the left portion of the window is a solid rest for the tip of the needle which projects from the transfixed mucosa prolapse. In this manner the tip of the needle remains in a position transverse to the wall of the valve and is therefore easily visible. It is therefore possible to remove the needle holder from the eye of the needle and, since this rests on the edge of the window with its tip pointing towards the centre of the rectal ampule, and grasp using the needle holder itself the easily visible tip, directly lit by the means of illumination, housed in the grip of the valve. In fact, the curved needle is solicited by the mucosa introflexed into the window. Should the tip of the needle not be resting on the edge of the window, the needle, due to its curved shape would be carried by the mucosa, sliding axially along the rectal ampule, in a sideways position, being half hidden by the mucosa irrigated by the blood which runs as it is transfixed. In this manner it is no longer necessary to use a further needle holder during the operation to grasp the tip of the needle without letting go of the eye, the procedure is therefore further simplified in an opening area which is by its very nature narrow.

It is also an advantage that the means of illumination are associated with the grip, making it possible to see the operating area clearly and at the same time not obstructing it.

A further advantage is that the window is elliptical or rhomboidal. This allows the mucosa, being longitudinal to the rectal ampule, to introflect spontaneously into the window in a sufficient quantity to easily create a purse-string suture in the position desired.

The fact that the tapering channel and the funnel-shaped section of the valve form divaricate the anal orifice sufficiently to create the necessary operating field is also an advantage.

It must also be appreciated that the rounded edges of the valve make it possible to avoid trauma during introduction into the active portion of the anal tract and the rectal ampule.

The ribs on the grip of the valve advantageously prevent it slipping when it is being manoeuvred.

The spatula with a grip angled with respect to the working section makes it possible to manoeuvre the spatula precisely and securely to co-operate operatively with the valve and obtain an optimal visual field.

The central section of the spatula being of limited bulk makes it possible to avoid obstructing the operative field. At the same time the active section of a width equal to the valve makes it possible to move the mucosa of the rectal ampule in front of the window of the valve from the operative field allowing the suturing of the mucosa.

During the operation the strangulation foreseen in the central section of the spatula is positioned in correspondence with the narrowing of the anal orifice. Thus the spatula is manoeuvred easily and traumatic action is avoided. The function of the spatula is in fact carried out deep in the intestinal tract, where it is necessary to remove from the operative field the mucosa and where the active portion of the spatula is of a width equal to the width of the valve.

A further advantage of the invention is that the set of accessories in synthetic material can be disposable or single-use.

## Claims

1. Value (20) for transanal operation, having an active section (22), wherein said active section (22) of said valve (20) is channel-shaped (24) and said channel (24) has an opening or window (34) to receive an introflexed part of mucosa (150), **characterized in that** said channel (24) has a semi-circular cross section and said opening or window (34) is positioned centrally to the channel (24) in the wall (30) of the valve(20).

2. Valve according to claim 1, **characterized in that** said opening (34) is positioned in proximity with an extremity (28) of the active section (22) of said valve (20).

3. Valve according to claim 1, **characterized in that** said valve (20) has an axis of symmetry (X-X) along which said opening (34) runs.

4. Valve according to claim 3, **characterized in that** said opening (34) is rhomboidal

5. Valve according to claim 3, **characterized in that** the edge (36) of said opening (34) has opposing sections (38, 40) positioned parallely to the axis of symmetry (X-X) of the valve (20).

6. Valve according to claim 1, **characterized in that** said channel (24) of the valve (20) tapers from a first extremity (26) to a second extremity (28).

7. Valve according to claim 1, **characterized in that** said active section (22) extends into a grip or handle (48).

8. Valve according to claim 7, **characterized in that** said active section (22) forms an angle (α) with said grip (48).

9. Valve according to claim 8, **characterized in that** said angle (α) formed between the active section (22) and the grip (48) of said valve (20) is between 100 degrees and 145 degrees and, is preferably equal to 120 degrees.

10. Valve according to claim 7, **characterized in that** said active section (22) of said valve (20) is joined to the grip (48) by a funnel-shaped section (52).

11. Valve according to claim 1, **characterized in that** on the back (42) of said active section (22) there is a graduated scale (44).

12. Valve according to claim 1, **characterized in that** said active section (22) of said valve (20) can be associated with an ogival inserter (69) for the introduction of the valve (20) into the rectal ampule (152).

13. Valve according to claim 7, **characterized in that** said grip (48) of said valve (20) can be associated with a means of illumination (64).

14. Valve according to claim 13, : **characterized in that** the grip is a tubular grip (48), which being hollow (62) houses a means of illumination (64).

15. Valve according to claim 14, **characterized in that** said channel has a tapered extremity (66) ending in said opening (68) in proximity with the extremity (26) of the active section (22).

16. Valve according to claim 13, **characterized in that** the bulb (70) of the means of illumination (64) juts out of said opening (68).

17. Valve according to claim 1, **characterized in that** said means of illumination (64) is fitted with hooks (71) which fit into a seat (73) on the external wall of the grip (48).

18. Valve according to claim 1, **characterized in that** said valve (20) is in biocompatible synthetic material, preferably of plastic of the type used for foodstuffs.

19. Valve according to claim 18, **characterized in that** said valve (20) is in ARL polystyrene, preferably transparent.

20. Set of accessories including at least one valve (20) as defined in claim 1, further including a spatula (74) with an active section (76) which joins a grip (78) at an angle (β).

21. Set of accessories, according to claim 20, **characterized in that** said angle (β) formed between the active section (76)and the grip (78) of said spatula (74) is between 100 degrees and 140 degrees and preferably equal to 120 degrees.

22. Set of accessories, according to claim 20, **characterized in that** said active section (76) of said spatula (74) is channel-shaped.

23. Set of accessories, according to claim 20, **characterized in that** said active section (76) of said spatula (74) has a width corresponding to the width of the active section (22) of the valve (20).

24. Set of accessories, according to claim 20, **characterized in that** said active section (76) of said spatula (74) is joined to the grip (78) by a section (82) having a width smaller than the width of the active section (76).

25. Set of accessories, according to claim 20, **characterized in that** said spatula (74) is in synthetic biocompatible material and preferably of the type used for foodstuffs.

26. Set of accessories, according to claim 20, **characterized in that** said spatula (74) is in ARL polystyrene and preferably transparent.

## Patentansprüche

1. Ventil (20) für transanale Eingriffe, mit einem aktiven Abschnitt (22), wobei der aktive Abschnitt (22) des Ventils (20) kanalförmig (24) ist und der Kanal (24) eine Öffnung oder ein Fenster (34) zur Aufnahme eines introflektierten Teils einer Schleimhaut (150) enthält, **dadurch gekennzeichnet, dass** de Kanal (24) einen halbkreisförmigen Querschnitt hat und die Öffnung bzw. das Fenster (34) zentral bezüglich des Kanals (24) in der Wandung (30) des Ventils (20) positioniert ist.

2. Ventil nach Anspruch 1, **dadurch gekennzeichnet, dass** die Öffnung (34) nahe einem äußersten Ende (28) des aktiven Abschnitts (22) des Ventils (20) angeordnet ist.

3. Ventil nach Anspruch 1, **dadurch gekennzeichnet, daß** das Ventil (20) eine Symmetrieachse (X-X) hat, entlang der die Öffnung (34) verläuft.

4. Ventil nach Anspruch 3, **dadurch gekennzeichnet, dass** die Öffnung (34) rhombusförmig ist.

5. Ventil nach Anspruch 3, **dadurch gekennzeichnet, dass** die Kante (36) der Öffnung (34) gegenüberliegende Teile (38, 40) hat, die parallel zur Symmetrieachse (X-X) des Ventils (20) liegen.

6. Ventil nach Anspruch 1, **dadurch gekennzeichnet, dass** der Kanal (24) des Ventils (20) sich von einem ersten äußersten Ende (26) zu einem zweiten äußersten Ende (28) hin verjüngt.

7. Ventil nach Anspruch 1, **dadurch gekennzeichnet, dass** der aktive Abschnitt (22) sich in einen (Hand-) Griff (48) hinein erstreckt.

8. Ventil nach Anspruch 7, **dadurch gekennzeichnet, dass** der aktive Abschnitt (22) mit dem Griff (48) einen Winkel (α) bildet.

9. Ventil nach Anspruch 8, **dadurch gekennzeichnet, dass** der zwischen dem aktiven Abschnitt (22) und dem Griff (48) des Ventils (20) gebildete Winkel (α) zwischen 100° und 145° beträgt und vorzugsweise gleich 120° ist.

10. Ventil nach Anspruch 7, **dadurch gekennzeichnet, dass** der aktive Abschnitt (22) des Ventils (20) über einen trichterförmigen Abschnitt (52) an den Griff (48) angefügt ist.

11. Ventil nach Anspruch 1, **dadurch gekennzeichnet, dass** sich auf dem Rücken (42) des aktiven Abschnitts (22) eine Skala (44) befindet.

12. Ventil nach Anspruch 1, **dadurch gekennzeichnet, dass** sich dem aktiven Abschnitt (22) des Ventils (20) ein ogivenförmiges Einführelement (69) zum Einführen des Ventils (20) in die Ampula recti (152) zuordnen lässt.

13. Ventil nach Anspruch 7, **dadurch gekennzeichnet, dass** sich dem Griff (48) des Ventils (20) eine Beleuchtungseinrichtung (64) zuordnen lässt.

14. Ventil nach Anspruch 13, **dadurch gekennzeichnet, dass** der Griff ein rohrförmiger Griff (48) ist, der, da er hohl (62) ist, eine Beleuchtungseinrichtung (64) aufnimmt.

15. Ventil nach Anspruch 14, **dadurch gekennzeichnet, dass** der Kanal ein verjüngtes äußeres Ende (66) hat, das nahe des äußersten Endes (26) des aktiven Abschnitts (22) zur Öffnung (68) ausläuft.

16. Ventil nach Anspruch 13, **dadurch gekennzeichnet, dass** die Lampe (70) der Beleuchtungseinrichtung (64) aus der Öffnung (68) hervorragt.

17. Ventil nach Anspruch 1, **dadurch gekennzeichnet, dass** die Beleuchtungseinrichtung (64) mit Haken (71) versehen ist, die in einen Sitz (73) auf der Außenwandfläche des Griffs (48) passen.

18. Ventil nach Anspruch 1, **dadurch gekennzeichnet, dass** das Ventil (20) aus einem biokompatiblen synthetischen Material und vorzugsweise aus einem Kunststoff der für Nahrungsmittel verwendeten Art besteht.

19. Ventil nach Anspruch 18, **dadurch gekennzeichnet, dass** das Ventil (20) aus ARL-Polystyrol besteht und vorzugsweise transparent ist.

20. Instrumentensatz mit mindestens einem Ventil (20) nach Anspruch 1, weiterhin mit einem Spatel (74) mit einem aktiven Abschnitt (76), der unter einem Winkel (β) an einen Griff (78) angefügt ist.

21. Instrumentensatz nach Anspruch 20, **dadurch gekennzeichnet, dass** der Winkel (β) zwischen dem aktiven Abschnitt (76) und dem Griff (78) zwischen 100° und 140° beträgt und vorzugsweise gleich 120° ist.

22. Instrumentensatz nach Anspruch 20, **dadurch gekennzeichnet, dass** der aktive Abschnitt (76) des Spatels (74) kanalförmig ist.

23. Instrumentensatz nach Anspruch 20, **dadurch gekennzeichnet, dass** der aktive Abschnitt (76) des Spatels (74) eine Breite entsprechend der Breite des aktiven Abschnitts (22) des Ventils (20) hat.

24. Instrumentensatz nach Anspruch 20, **dadurch gekennzeichnet, dass** der aktive Abschnitt (76) des Spatels (74) an den Griff (78) mit einem Abschnitt (82) angefügt ist, dessen Breite geringer ist als die des aktiven Abschnitts (76).

25. Instrumentensatz nach Anspruch 20, **dadurch gekennzeichnet, dass** der Spatel (74) aus einem biokompatiblen synthetischen Material und vorzugsweise der für Nahrungsmittel verwendeten Art besteht.

26. Instrumentensatz nach Anspruch 20, **dadurch gekennzeichnet, dass** der Spatel (74) aus ARL-Polystyrol besteht und vorzugsweise transparent ist.

## Revendications

1. Valve (20) pour opération transanale, ayant une section active (22), dans laquelle ladite section active (22) de ladite valve (20) est en forme de canal (24) et ledit canal (24) comporte une ouverture ou fenêtre (34) destinée à recevoir une partie de muqueuse en flexion interne (150), **caractérisée en ce que** ledit canal (24) a une section transversale semi-circulaire et ladite ouverture ou fenêtre (34) est positionnée en position centrale par rapport au canal (24) dans la paroi (30) de la valve (20).

2. Valve selon la revendication 1, **caractérisée en ce que** ladite ouverture (34) est positionnée à proximité d'une extrémité (28) de la section active (22) de ladite valve (20).

3. Valve selon la revendication 1, **caractérisée en ce que** ladite valve (20) a un axe de symétrie (X-X) le long duquel s'étend ladite ouverture (34).

4. Valve selon la revendication 3, **caractérisée en ce que** ladite ouverture (34) est rhomboïde.

5. Valve selon la revendication 3, **caractérisée en ce que** le bord (36) de ladite ouverture (34) possède des sections opposées (38, 40) positionnées parallèlement à l'axe de symétrie (X-X) de la valve (20).

6. Valve selon la revendication 1, **caractérisée en ce que** ledit canal (24) de la valve (20) s'amincit d'une première extrémité (26) à une seconde extrémité (28).

7. Valve selon la revendication 1, **caractérisée en ce que** ladite section active (22) s'étend dans une poignée ou un manche (48).

8. Valve selon la revendication 7, **caractérisée en ce que** ladite section active (22) forme un angle (α) avec ladite poignée (48).

9. Valve selon la revendication 8, **caractérisée en ce que** ledit angle (α) formé entre la section active (22) et la poignée (48) de ladite valve (20) est compris entre 100 degrés et 145 degrés et est de préférence égal à 120 degrés.

10. Valve selon la revendication 7, **caractérisée en ce que** ladite section active (22) de ladite valve (20) est assemblée à la poignée (48) par une section en forme d'entonnoir (52).

11. Valve selon la revendication 1, **caractérisée en ce que**, à l'arrière (42) de ladite section active (22), se trouve une échelle graduée (44).

12. Valve selon la revendication 1, **caractérisée en ce que** ladite section active (22) de ladite valve (20) peut être associée à un élément d'introduction ogival (69) pour l'introduction de la valve (20) dans l'ampoule rectale (152).

13. Valve selon la revendication 7, **caractérisée en ce que** ladite poignée (48) de ladite valve (20) peut être associée à des moyens d'éclairage (64).

14. Valve selon la revendication 13, **caractérisée en ce que** la poignée est une poignée tubulaire (48) qui, étant creuse (62), renferme des moyens d'éclairage (64).

15. Valve selon la revendication 14, **caractérisée en ce que** ledit canal possède une extrémité amincie (66) se terminant dans ladite ouverture (68) à proximité de l'extrémité (26) de la section active (22).

16. Valve selon la revendication 13, **caractérisée en ce que** l'ampoule (70) des moyens d'éclairage (64) fait saillie de ladite ouverture (68).

17. Valve selon la revendication 1, **caractérisée en ce que** lesdits moyens d'éclairage (64) sont fixés par des crochets (71) qui s'insèrent dans un siège (73) sur la paroi externe de la poignée (48).

18. Valve selon la revendication 1, **caractérisée en ce que** ladite valve (20) est en matériau synthétique biocompatible, de préférence en plastique du type utilisé pour les aliments.

19. Valve selon la revendication 18, **caractérisée en ce que** ladite valve (20) est en polystyrène ARL, de préférence transparente.

20. Ensemble d'accessoires comprenant au moins une valve (20) selon la revendication 1, comprenant en outre une spatule (74) avec une section active (76) qui rejoint une poignée (78) à un angle (β).

21. Ensemble d'accessoires selon la revendication 20, **caractérisé en ce que** ledit angle (β) formé entre la section active (76) et la poignée (78) de ladite spatule (74) est compris entre 100 degrés et 140 degrés et est de préférence égal à 120 degrés.

22. Ensemble d'accessoires selon la revendication 20, **caractérisé en ce que** ladite section active (76) de ladite spatule (74) est en forme de canal.

23. Ensemble d'accessoires selon la revendication 20, **caractérisé en ce que** ladite section active (76) de ladite spatule (74) a une largeur correspondant à la largeur de la section active (22) de la valve (20).

24. Ensemble d'accessoires selon la revendication 20, **caractérisé en ce que** ladite section active (76) de ladite spatule (74) est assemblée à la poignée (78) par une section (82) ayant une largeur plus petite que la largeur de la section active (76).

25. Ensemble d'accessoires selon la revendication 20, **caractérisé en ce que** ladite spatule (74) est en matériau biocompatible synthétique et de préférence du type utilisé pour les aliments.

26. Ensemble d'accessoires selon la revendication 20, **caractérisé en ce que** ladite spatule (74) est en polystyrène ARL et de préférence transparent.
